# EUROPEAN PATENT SPECIFICATION

(11) **EP 2 539 001 B1**
(45) Date of publication and mention of the grant of the patent: **13.12.2017**
(21) Application number: 11706698.5
(22) Date of filing: 18.02.2011
(51) Int. Cl.: A61B 5/0205, A61B 5/024, A61B 5/08, A61B 5/091, A61B 5/1455, A61M 16/00

(54) **SPONTANEOUS BREATHING TRIAL MANAGER**
TESTMANAGER FÜR SPONTANE ATMUNG
GESTIONNAIRE DE TEST DE RESPIRATION SPONTANÉE

(30) Priority: 26.02.2010 US 714248
(43) Date of publication of application: 02.01.2013
(73) Proprietor: Covidien LP, Mansfield, MA 02048 (US)
(72) Inventor: DOYLE, Peter, Vista, California 92081 (US); VANDINE, Joseph, Manteca, California 95337 (US)
(74) Representative: Hargreaves, Timothy Edward
(86) International application number: PCT/US2011/025370
(87) International publication number: WO 2011/106249

(56) References cited:
- WO-A1-2009/043144
- WO-A1-2009/120607
- US-A- 6 148 814
- US-B1- 6 273 088

## Description

### Introduction

Medical ventilator systems have been long used to provide supplemental breathing support to patients. These ventilators typically comprise a source of pressurized gas which is fluidly connected to the patient through a conduit. In some systems, the patient after an extended period of ventilation is placed on spontaneous breathing trials (SBT). The spontaneous breathing trials help to determine whether the patient is ready to be weaned from ventilator support.

The SBT is often conducted at low levels of ventilator support for a varying and/or constant period of time. The patient typically remains on the ventilator during the SBT to allow for better monitoring (of their tolerance of the SBT). The bedside clinician sets the breathing mode, spontaneous breath type and all associated settings for the SBT (either under a protocol or on the order of a physician).

However, there may be occasions where the bedside clinician cannot remain at the bedside for the duration of the set SBT time interval or cannot immediately attend to the patient if the patient has exceeded limits of monitored variables indicating a failure of the trial. Accordingly, conducting a SBT inconveniently require the bedside clinician to remain with the patient or be available to the patient for the duration of the SBT interval.

US 6,148,814 discloses a system for managing ventilation of a patient and for updating an administrative database with relevant data of the patient. WO 2009/120607 A1 discloses a method for controlling delivery of breathing gas to a patient using multiple ventilation parameters. The prior art also comprises US 6,273,088 B1, which describes an apparatus and method to monitor patient breathing during weaning from ventilator dependency, and WO 2009/120707, which describes a method for controlling the delivery of a breathing gas to a patient.

### Summary

The invention is defined in the independent claim. This disclosure describes systems and methods for conducting and terminating spontaneous breathing trials on patients receiving mechanical ventilation. The disclosure describes a novel spontaneous breathing trial manager for a medical ventilator with rapid initiation and continuous monitoring of a patient's tolerance of the spontaneous breathing trial and displaying of that tolerance as a function of time, which provides for bedside adjustment of the spontaneous breathing trial parameters and automatic termination of a spontaneous breathing trial based on a time interval expiration or poor patient tolerance of the SBT.

This disclosure describes a method for managing a spontaneous breathing trial in a medical ventilator. The method includes performing the following steps:
a) initiating a spontaneous breathing trial for a patient being ventilated on a medical ventilator;
b) monitoring a plurality of sensors to obtain a plurality of sensor measurements during the spontaneous breathing trial;
c) determining whether at least one of the plurality of sensor measurements is outside of a desired range for a predetermined amount of time;
d) determining whether a RSBI calculation is outside of a desired range for a predetermined amount of time;
e) ending the spontaneous breathing trial based on at least one of a determination that at least one of the plurality of sensor measurements is outside of the desired range for the predetermined amount of time, the RSBI calculation is outside of the desired range for the predetermined amount of time, an inputted user command, and expiration of a spontaneous breathing trial period;
f) displaying at least one of the plurality of sensor measurements as a function of time for the spontaneous breathing trial; and
g) displaying a basis for the step of ending the spontaneous breathing trial for the patient being ventilated on the medical ventilator.

This disclosure also describes a medical ventilator system including: a processor; a gas regulator controlled by the processor, the gas regulator adapted to regulate a flow of gas from a gas supply to a patient via a patient circuit; a breath frequency sensor controlled by the processor, the breath frequency sensor is adapted to measure the breath frequency of the patient; a spontaneous tidal volume sensor controlled by the processor, the spontaneous tidal volume sensor is adapted to measure spontaneous tidal volume of the patient; a spontaneous exhalation volume sensor controlled by the processor, the spontaneous exhalation volume sensor is adapted to measure spontaneous exhalation volume of the patient; a SpO₂ sensor controlled by the processor, the SpO₂ sensor is adapted to measure blood oxygen saturation level of the patient; a heart rate sensor controlled by the processor, the heart rate sensor is adapted to measure heart rate of the patient; a spontaneous breathing trial manager in communication with the processor, the breath frequency sensor, the spontaneous tidal volume sensor, the spontaneous exhalation volume sensor, the SpO₂ sensor, and the heart rate sensor; a user interface in communication with the processor and the spontaneous breathing trial manager; and a display module controlled by the processor, the display module adapted to display RSBI and at least one of heart rate, blood oxygen saturation level, spontaneous tidal volume, and spontaneous exhalation volume of the patient as a function of time for a spontaneous breathing trial. The spontaneous breathing trial manager further includes a threshold monitor module and a ventilation module.

Yet, another aspect of the disclosure describes a pressure support system. The pressure support system includes: a processor; a pressure generating system adapted to generate a flow of breathing gas controlled by the processor; a ventilation system including a patient circuit controlled by the processor; a breath frequency sensor controlled by the processor, the breath frequency sensor is adapted to measure breath frequency of the patient; a spontaneous tidal volume sensor controlled by the processor, the spontaneous tidal volume sensor is adapted to measure spontaneous tidal volume of the patient; a spontaneous exhalation volume sensor controlled by the processor, the spontaneous exhalation volume sensor is adapted to measure spontaneous exhalation volume of the patient; a SpO₂ sensor controlled by the processor, the SpO₂ sensor is adapted to measure blood oxygen saturation level of the patient; a heart rate sensor controlled by the processor, the heart rate sensor is adapted to measure heart rate of the patient; a spontaneous breathing trial manager in communication with the processor, the breath frequency sensor, the spontaneous tidal volume sensor, the spontaneous exhalation volume sensor, the SpO₂ sensor, and the heart rate sensor; a user interface in communication with the processor and the spontaneous breathing trial manager; and a display module controlled by the processor, the display module adapted to display heart rate, RSBI, blood oxygen saturation level, spontaneous tidal volume, and spontaneous exhalation volume of the patient as a function of time for a spontaneous breathing trial. The spontaneous breathing trial manager further includes a threshold monitor module and a ventilation module.

These and various other features as well as advantages will be apparent from a reading of the following detailed description and a review of the associated drawings. Additional features are set forth in the description that follows and, in part, will be apparent from the description, or may be learned by practice of the described embodiments. The benefits and features will be realized and attained by the structure particularly pointed out in the written description and claims hereof as well as the appended drawings.

It is to be understood that both the foregoing general description and the following detailed description are exemplary and explanatory and are intended to provide further explanation of the claimed invention.

### Brief Description of the Drawings

The following drawing figures, which form a part of this application, are illustrative of embodiments systems described below and are not meant to limit the scope of the invention in any manner, which scope shall be based on the claims appended hereto.
FIG. 1 illustrates an embodiment of a ventilator connected to a human patient.
FIG. 2 illustrates an embodiment of an operatively coupled ventilator, spontaneous breathing trial manager, and display.
FIG. 3 illustrates an embodiment of a spontaneous breathing trial method for a medical ventilator.
FIG. 4 illustrates an embodiment of a display screen shot for a spontaneous breathing trial listing the ventilator parameters of a spontaneous breathing trial and user interface commands.
FIG. 5 illustrates an embodiment of a display screen shot for a spontaneous breathing trial on a medical ventilator graphing key patient variables verses time for the spontaneous breathing trial.
FIG. 6 illustrates an embodiment of a display screen shot for a spontaneous breathing trial on a medical ventilator graphing key patient variables verses time for the spontaneous breathing trial and the cause for ending the spontaneous breathing trial.

### Detailed Description

Although the techniques introduced above and discussed in detail below may be implemented for a variety of medical devices, the present disclosure will discuss the implementation of these techniques in the context of a medical ventilator for use in providing ventilation support to a human patient. The reader will understand that the technology described in the context of a medical ventilator for human patients could be adapted for use with other systems such as ventilators for non-human patients and general gas transport systems in which periodic gas mixture changes may be required. As utilized herein a "gas mixture" includes at least one of a breathing gas and a mixture of breathing gases.

Medical ventilators are used to provide a breathing gas to a patient who may otherwise be unable to breathe sufficiently. In modern medical facilities, pressurized air and oxygen sources are often available from wall outlets. Accordingly, ventilators may provide pressure regulating valves (or regulators) connected to centralized sources of pressurized air and pressurized oxygen. The regulating valves function to regulate flow so that respiratory gas having a desired concentration of oxygen and other gases is supplied to the patient at desired pressures and rates. Ventilators capable of operating independently of external sources of pressurized air are also available.

While operating a ventilator, it can be desirable to provide spontaneous breathing trials (SBTs) that do not require the clinician to be present at the end of the set SBT time interval or available in case the patient exceeds a key variable threshold during the SBT.

Accordingly, a SBT manager for rapid initiation of SBTs (using institution-configured setting with flexibility for bedside adjustment, including desired duration) that monitors key variables to determine the patient's tolerance to the SBTs for a medical ventilator is desirable. The SBT manager automatically returns a patient to the previous (prior to SBT) ventilator settings in the event the preset time has elapsed or the patient has exceeded a clinician-set monitored variable thresholds. Further, the SBT manager records the trend of the patient's progress during the SBT and any causes for resumption of the previous setting, if this occurred for clinician review.

The SBT manager provides for several advantages. In one embodiment, the SBT manager improves the ease of use of the ventilator and a SBT. In a further embodiment, the SBT manager decreases the amount of time a clinician must monitor a patient during a SBT than previously utilized SBT ventilator systems. In another embodiment, the SBT manager decreases the amount of time necessary to program and/or initiate a SBT by a clinician than previously utilized SBT ventilator systems. In an additional embodiment, the SBT manager provides for better ventilator adherence to protocols than previously utilized SBT ventilator systems.

Those skilled in the art will recognize that the methods and systems of the present disclosure may be implemented in many manners and as such are not to be limited by the foregoing exemplary embodiments and examples. In other words, functional elements being performed by a single or multiple components, in various combinations of hardware and software or firmware, and individual functions, can be distributed among software applications, which may be distributed among one or multiple processors. In this regard, any number of the features of the different embodiments described herein may be combined into single or multiple embodiments, and alternate embodiments having fewer than or more than all of the features herein described are possible. Functionality may also be, in whole or in part, distributed among multiple components, in manners now known or to become known. Thus, myriad software/hardware/firmware combinations are possible in achieving the functions, features, interfaces and preferences described herein. Moreover, the scope of the present disclosure covers conventionally known manners for carrying out the described features and functions and interfaces, and those variations and modifications that may be made to the hardware or software or firmware components described herein as would be understood by those skilled in the art now and hereafter.

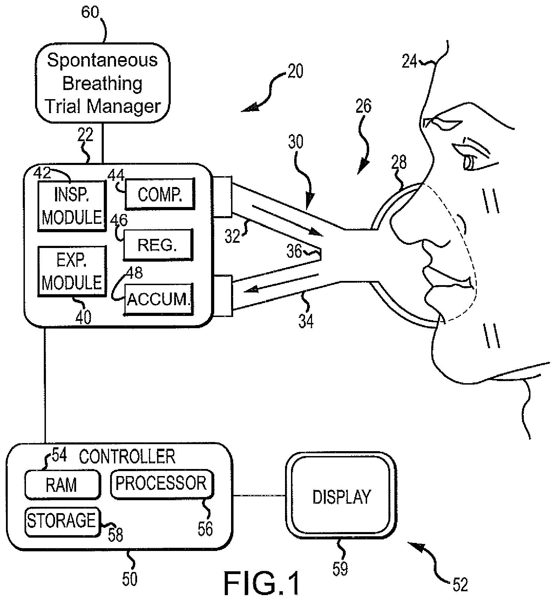

FIG. 1 illustrates an embodiment of a ventilator **20** connected to a human patient **24.** Ventilator **20** includes a pneumatic system **22** (also referred to as a pressure generating system **22**) for circulating breathing gases to and from patient **24** via the ventilation tubing system **26,** which couples the patient **24** to the pneumatic system **22** via physical patient interface **28** and ventilator circuit **30.** Ventilator circuit **30** could be a two-limb or one-limb circuit for carrying gas mixture to and from the patient **24.** In a two-limb embodiment as shown, a wye fitting **36** may be provided to couple the patient interface **28** to the inspiratory limb **32** and the expiratory limb **34** of the circuit **30.**

The present systems and methods have proved particularly advantageous in invasive settings, such as with endotracheal tubes. However, condensation and mucus buildup do occur in a variety of settings, and the present description contemplates that the patient interface **28** may be invasive or non-invasive, and of any configuration suitable for communicating a flow of breathing gas from the patient circuit **30** to an airway of the patient **24.** Examples of suitable patient interface **28** devices include a nasal mask, nasal/oral mask (which is shown in FIG. 1), nasal prong, full-face mask, tracheal tube, endotracheal tube, nasal pillow, etc.

Pneumatic system **22** may be configured in a variety of ways. In the present example, system **22** includes an expiratory module **40** coupled with an expiratory limb **34** and an inspiratory module **42** coupled with an inspiratory limb **32.** Further, the gas concentrations can be mixed and/or stored in a chamber of a gas accumulator **44** at a high pressure to improve the control of delivery of respiratory gas to the ventilator circuit **30.** The inspiratory module **42** is coupled to the gas regulator **46** and accumulator **44** to control the gas mixture of pressurized breathing gas for ventilatory support via inspiratory limb **32.**

The pneumatic system **22** may include a variety of other components, including other sources for pressurized air and/or oxygen, mixing modules, valves, sensors, tubing, filters, etc. In one embodiment, the pneumatic system 22 includes at least one of a breathing frequency sensor, a spontaneous tidal volume (V_{t spont}) sensor, a spontaneous exhalation volume (V_{e spont}) sensor, a carbon dioxide elimination sensor, a SpO₂ sensor, and a heart rate sensor. In another embodiment, the pneumatic system 22 includes a breath frequency sensor and at least one of a spontaneous tidal volume (V_{t spont}) sensor, a spontaneous exhalation volume (V_{e spont}) sensor, a carbon dioxide elimination sensor, a blood oxygen saturation level (SpO₂) sensor, and a heart rate sensor.

As shown, ventilator **20** further includes a spontaneous breathing trial manager **60** operatively coupled to the controller **50** and the pneumatic system **22.** In one embodiment, the spontaneous breathing trial manager **60** is a separate independent component from ventilator **20.** In an alternative embodiment, the spontaneous breathing trial manager **60** is incorporated in pneumatic system **22.**

The spontaneous breathing trial manager **60** initiates a spontaneous breathing trial based on preset configurations, inputted command, or a selected mode. The SBT manager **60** provides for rapid initiation of SBTs (using institution- or factory-configured settings with flexibility for bedside adjustment, including desired duration) that monitors key variables to determine the patient's tolerance of the SBTs. In one embodiment, the key variables include at least one of a ratio of respiratory frequency in respirations per minute to tidal volume in liters (f/Vₜ) or as otherwise known as a rapid shallow breathing index (RSBI), spontaneous tidal volume (V_{t spont}), spontaneous exhalation volume (V_{e spont}), carbon dioxide elimination levels, blood oxygen saturation level (SpO₂), heart rate and the patient's breathing work estimate. The RSBI is calculated by utilizing an algorithm run by the processor. In another embodiment, the key variables include the ratio of respiratory frequency in respirations per minute to tidal volume in liters (f/Vₜ) or rapid shallow breathing index (RSBI) and at least one of spontaneous tidal volume (V_{t spont}), spontaneous exhalation volume (V_{e spont}), carbon dioxide elimination levels, blood oxygen saturation level (SpO₂), heart rate and the patient's breathing work estimate. The patient's breathing work estimate is determined when the ventilator is in a proportional assist ventilation mode or option. The SBT manager **60** automatically returns a patient **24** to the previous (prior to SBT) ventilator settings in the event the preset time has elapsed or the patient **24** has exceeded the clinician-set monitored variable thresholds. Further, the SBT manager **60** records the trend of the patient's progress during the SBT and any causes for resumption of the previous setting, if this occurred for clinician review. In one embodiment, the SBT manager **60** sends the patient's progress during the SBT to the display **59** for user viewing.

In the illustrated embodiment, ventilator **20** includes a display **59.** The SBT manager **60** is operatively coupled to the ventilator display **59.** In an alternative embodiment, the SBT manager **60** is operatively coupled to a separate display **59** component that is independent of the SBT manger **60** and the ventilator **20.** In another embodiment, the SBT manager **60** includes a display **59.**

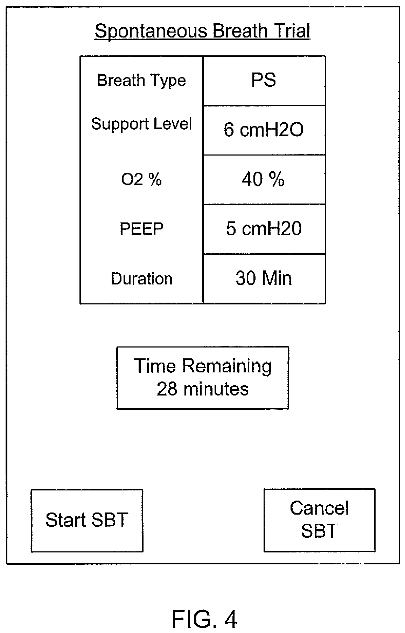

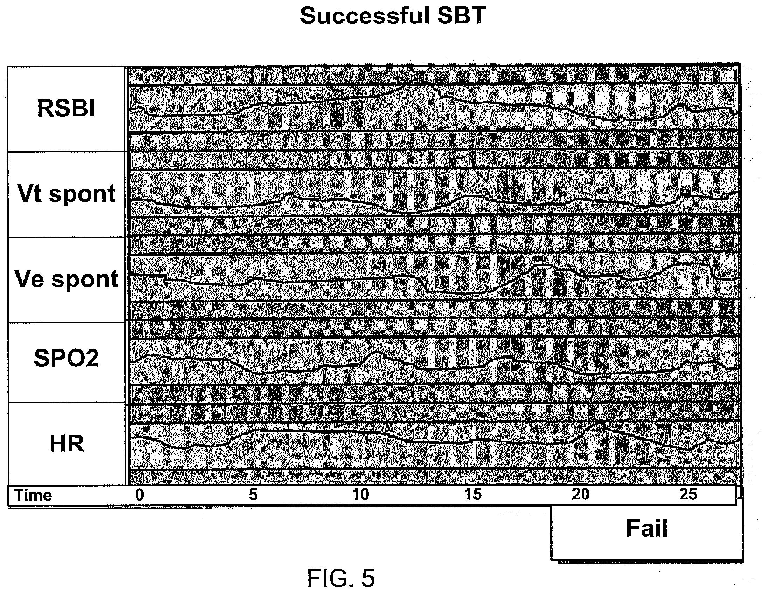

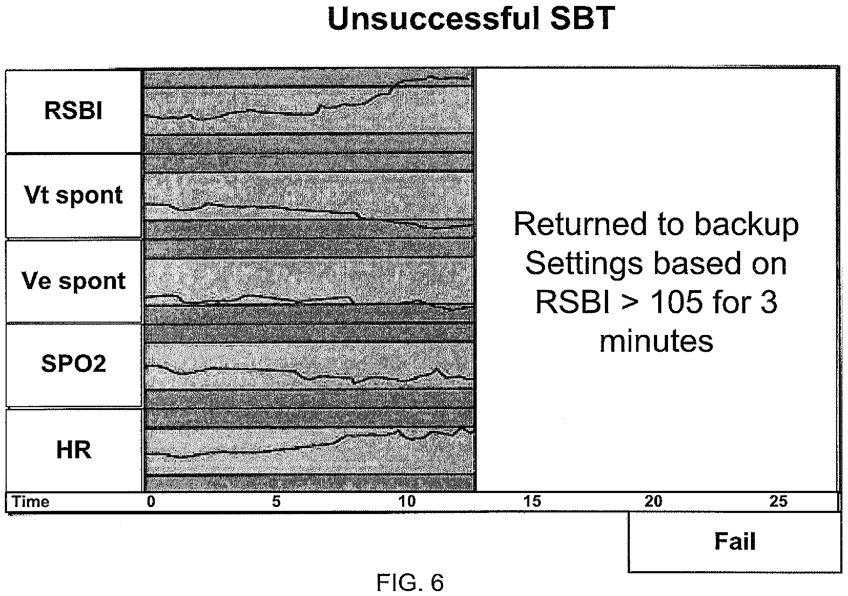

The display **59** can display any type of ventilation, patient, or SBT manager information, such as sensor readings, parameters, commands, alarms, warnings, and smart prompts (i.e., ventilator determined operator suggestions). In one embodiment, the display **59** lists the breath type utilized by ventilator **20,** the pressure support level, the percentage of oxygen in the gas mixture, the positive end-expiratory pressure (PEEP), the predetermined amount of time for the SBT trial, and the amount of time remaining of the SBT period, as illustrated in FIG. 4. In another embodiment, the display **59** may show the trend of the patient's progress as a function of time during the SBT, as illustrated in FIGS. 5 and 6. In one embodiment, the display illustrates at least one of a RSBI calculation, a spontaneous tidal volume measurement (V_{t spont}), a spontaneous exhalation volume (V_{e spont}) measurement, a carbon dioxide elimination measurement, a SpO₂ measurement, patient's breathing work estimate, and a heart rate measurement as a function of time. In another embodiment, the display illustrates the RSBI calculation and at least one of a spontaneous tidal volume measurement (V_{t spont}), a spontaneous exhalation volume (V_{e spont}) measurement, a carbon dioxide elimination measurement, a SpO₂ measurement, patient's breathing work estimate, and a heart rate measurement as a function of time. Further, in the depicted example, the display **59** includes an operator interface **52** that is touch-sensitive, enabling the display **59** to serve both as an input user interface and an output device.

Controller **50** is operatively coupled with pneumatic system **22,** SBT manager **60** signal measurement and acquisition systems, and an operator interface **52** may be provided to enable an operator to interact with the ventilator **20** (e.g., change ventilator settings, select operational modes, view monitored parameters, etc.). Controller **50** may include memory **54,** one or more processors **56,** storage **58,** and/or other components of the type commonly found in command and control computing devices.

The memory **54** is non-transitory computer-readable storage media that stores software that is executed by the processor **56** and which controls the operation of the ventilator **20.** In an embodiment, the memory **54** comprises one or more solid-state storage devices such as flash memory chips. In an alternative embodiment, the memory **54** may be mass storage connected to the processor **56** through a mass storage controller (not shown) and a communications bus (not shown). Although the description of non-transitory computer-readable media contained herein refers to a solid-state storage, it should be appreciated by those skilled in the art that non-transitory computer-readable storage media can be any available media that can be accessed by the processor **56.** Non-transitory computer-readable storage media includes volatile and non-volatile, removable and non-removable media implemented in any method or technology for storage of information such as non-transitory computer-readable instructions, data structures, program modules or other data. Non-transitory computer-readable storage media includes, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by the processor **56.**

In another embodiment, the program may be run in working memory or working volatile memory. The working volatile memory must be reloaded at each initiation and may consist of RAM, DRAM, SDRAM, and selected mainly for speed of access and execution.

The controller **50** issues commands to pneumatic system **22** in order to control the breathing assistance provided to the patient **24** by the ventilator **20.** The commands may be based on inputs received from patient **24,** pneumatic system **22** and sensors, operator interface **52,** SBT manager **60,** and/or other components of the ventilator **20.**

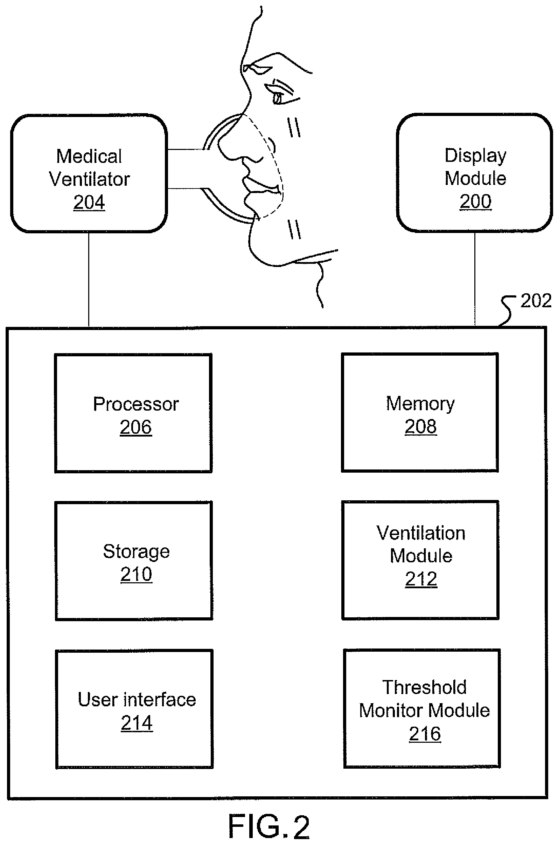

FIG. 2 illustrates an embodiment of a spontaneous breathing trial manager **202** (SBT manager **202**) operatively coupled with a medical ventilator **204** and a display module **200.** SBT manager **202** may include memory **208,** one or more processors **206,** storage **210,** and/or other components of the type commonly found in command and control computing devices.

The memory **208** is non-transitory computer-readable storage media that stores software that is executed by the processor **206** to determine commands to send to the ventilator **204** for controlling the ventilator settings. In an embodiment, the memory **208** comprises one or more solid-state storage devices such as flash memory chips. In an alternative embodiment, the memory **208** may be mass storage connected to the processor **206** through a mass storage controller (not shown) and a communications bus (not shown). Although the description of non-transitory computer-readable media contained herein refers to a solid-state storage, it should be appreciated by those skilled in the art that non-transitory computer-readable storage media can be any available media that can be accessed by the processor **206.** Non-transitory computer-readable storage media includes volatile and non-volatile, removable and non-removable media implemented in any method or technology for storage of information such as non-transitory computer-readable instructions, data structures, program modules or other data. Non-transitory computer-readable storage media includes, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by the processor **206.**

In an embodiment, the SBT manager **202** sends commands to the ventilator **204** or to the pneumatic system of the ventilator **204** in order to control ventilator settings. In another embodiment, a SBT manager **202** provides for quick set-up and rapid initiation of SBTs (using institution-configured setting with flexibility for bedside adjustment, including the predetermined amount of time for the SBT) that monitors key variables to determine the patient's tolerance to the SBTs for a medical ventilator **204.**

In one embodiment, the SBT manager **202** monitors key variables by receiving sensor measurements. In another embodiment, the SBT manager **202** monitors key variable by communicating with the processor. The processor may monitor the key variables by receiving sensor measurements. In one embodiment, the medical ventilator **204** includes at least one of a breath frequency sensor, a spontaneous tidal volume (V_{t spont}) sensor, a spontaneous exhalation volume (V_{e spont}) sensor, a carbon dioxide elimination sensor, a blood oxygen saturation level (SpO₂) sensor, patient's breathing work estimate, and heart rate sensor. In another embodiment, the medical ventilator **204** includes a rapid shallow breathing index (RSBI) monitor and at least one of a breath frequency sensor, a spontaneous tidal volume (V_{t spont}) sensor, a spontaneous exhalation volume (V_{e spont}) sensor, a carbon dioxide elimination sensor, a blood oxygen saturation level (SpO₂) sensor, and heart rate sensor. In another embodiment, the medical ventilator **204** includes a rapid shallow breathing index (RSBI) monitor, a breath frequency sensor, a spontaneous tidal volume (V_{t spont}) sensor, a spontaneous exhalation volume (V_{e spont}) sensor, a blood oxygen saturation level (SpO₂) sensor, and heart rate sensor.

Any ventilator parameter suitable for affecting a SBT may be adjusted by a user through the SBT manger **202** during a SBT. In one embodiment, the support level, the oxygen percentage of the gas mixture, PEEP, trial time period, and/or breath type of the ventilator can be adjusted by a user through the SBT manager **202.**

In one embodiment, the SBT manager **202** automatically returns a patient to the previous (prior to SBT) ventilator settings in the event the predetermined time has elapsed or the patient has exceeded the clinician-set monitored variable thresholds. Accordingly, the SBT manager **202** decreases the amount of time a clinician must monitor a patient during a SBT compared to previously utilized SBT ventilator systems. Further, the SBT manager **202** provides for better ventilator adherence to protocols than previously utilized SBT ventilator systems.

Additionally, the SBT manager **202** records the trend of the patient's progress during the SBT and any causes for resumption of the previous setting, if this occurred for clinician review.

As shown, the SBT manager **202** is operatively coupled to a separate and independent display module **200.** In an alternative embodiment, the display module **200** is incorporated in the ventilator or SBT manager **202.** The display module **200** is suitable for displaying ventilator information, patient information, and/or SBT information. In one embodiment, the display lists the breath type, support level, oxygen percentage of the gas mixture, PEEP, time period, and/or the time remaining of the SBT period, as illustrated in FIG. 4.

In one embodiment, the display module **200** is touch-sensitive, enabling the display to serve both as an input user interface and an output device. The user interface **214** allows a user to input commands, patient information, ventilator parameters, and SBT parameters. In one embodiment, the user interface **214** allows a user to start a SBT or cancel an already occurring SBT, as illustrated in FIG. 4. In another embodiment, the user interface **214** in the interactive display allows a user to change the predetermined amount of time for the SBT during a SBT period. Accordingly, the SBT manager **202** improves the ease of use of the ventilator and a SBT compared to previously utilized SBT systems.

In a further embodiment, the display module **200** illustrates the trend of the patient's progress during the SBT and any causes for resumption of the previous setting, if this occurred for clinician review. In one embodiment, the display graphically depicts a patient's progress during the SBT as a function of time for the SBT period. The patient's progress may be determined by monitoring different sensor measurements. In one embodiment, the patient's progress during the SBT is depicted by showing the rapid shallow breathing index (RSBI), respiration rate, spontaneous tidal volume (V_{t spont}) spontaneous exhalation volume (V_{e spont}), blood oxygen saturation level (SpO₂), and heart rate as a function of time, as illustrated in FIGS. 5 and 6. In another embodiment, the display illustrates at least one of a ratio of respiratory frequency in respirations per minute to tidal volume in liters (f/Vₜ), a carbon dioxide elimination level, a rapid shallow breathing index (RSBI), a respiration rate, a breathing work estimate, a spontaneous tidal volume (V_{t spont}), a spontaneous exhalation volume (V_{e spont}), a blood oxygen saturation level (SpO₂), and a heart rate as a function of time. In another embodiment, the display illustrates at least one of a ratio of respiratory frequency in respirations per minute to tidal volume in liters (f/Vₜ) or a RSBI and at least one of a carbon dioxide elimination level, a rapid shallow breathing index (RSBI), a respiration rate, a spontaneous tidal volume (V_{t spont}), a spontaneous exhalation volume (V_{e spont}), a blood oxygen saturation level (SpO₂), and a heart rate as a function of time.

As illustrated in FIG. 6, the reason for a failed SBT trial is shown on the display. In this embodiment, the RSBI exceeded the desired range for three minutes and the spontaneous tidal volume is below the desired range for a period of time; therefore, the SBT manager **202** terminated the SBT. In another embodiment, the SBT manager ended the SBT because RSBI and at least one of a carbon dioxide elimination measurement, a respiration rate measurement, a spontaneous tidal volume (V_{t spont}) measurement, a breathing work estimate, a spontaneous exhalation volume (V_{e spont}) measurement, a blood oxygen saturation level (SpO₂) measurement, and a heart rate measurement is outside of a desired range for a period of time. In a further embodiment, the SBT manager ended the SBT because RSBI is outside the desired range for three minutes and at least one of a carbon dioxide elimination measurement, a respiration rate measurement, a spontaneous tidal volume (V_{t spont}) measurement, a spontaneous exhalation volume (V_{e spont}) measurement, a blood oxygen saturation level (SpO₂) measurement, and a heart rate measurement is outside of a desired range for 5 seconds. In another embodiment, at least one of a ratio of respiratory frequency in respirations per minute to tidal volume in liters (f/Vₜ), a carbon dioxide elimination level, a rapid shallow breathing index (RSBI), a respiration rate, a spontaneous tidal volume (V_{t spont}), a spontaneous exhalation volume (V_{e spont}), a blood oxygen saturation level (SpO₂), and a heart rate are outside of their desired threshold for a period of time. In another embodiment, at least two of a carbon dioxide elimination measurement, a respiration rate measurement, a spontaneous tidal volume (V_{t spont}) measurement, a spontaneous exhalation volume (V_{e spont}) measurement, a blood oxygen saturation level (SpO₂) measurement, and a heart rate measurement are outside of their desired range for period time, such as three minutes. These embodiments are not limiting. Any suitable combination of exceeded parameters for any suitable period of time can be utilized to terminate a SBT. Further, any reason for termination of a SBT may be shown on the display monitor.

In the embodiment shown, the SBT manager **202** further includes a ventilation module **212,** a user interface **214,** and a threshold monitor module **216.** The threshold monitor module **216** utilizes ventilator and patient information to monitor the patient's tolerance of the SBTs for the medical ventilator **204.** The threshold monitor module **216** determines if key variables are within a desired range or beyond a desired threshold or range. The key variable may be monitored through sensor measurements. In one embodiment, the threshold monitor module **216** determines if key variables are within a desired range or beyond a desired threshold for a predetermined amount of time. The key variables are any suitable ventilator or patient information that is an indicator of the patient's tolerance to the SBT. In one embodiment, the key variables include the ratio of respiratory frequency in respirations per minute to tidal volume in liters (f/Vₜ), rapid shallow breathing index (RSBI), spontaneous tidal volume (V_{t spont}) spontaneous exhalation volume (V_{e spont}), blood oxygen saturation level (SpO₂), carbon dioxide elimination levels (V_{CO2}), and/or heart rate. Each key variable has a desired range for the patient during a SBT. One embodiment of desired thresholds for a patient during a SBT is illustrated in Table. 1 below:

**Table 1. Example Thresholds for Key Variables During a SBT**

| **Key Variable** | **Threshold** |
|---|---|
| Respiration Rate | > 35 breaths per min for a period of 5 minutes |
| | to |
| | < 8 breaths per minute for a period of greater than 30 seconds |
| SpO₂ | < 90% O₂ for a period of 3 minutes |
| Heart Rate | > 130 beats per minute |
| | or |
| | a heart beat changes of 20% |
| RSBI | > 105 |
| V_{CO2} | < 150 mL/min |
| | or |
| | < 85% of V_{CO2} prior to start of SBT |
| | or |
| | an increase of V_{CO2} > 25% over the V_{CO2} prior to the start of the SBT |
| V_{t spont} | < 3.5 mL/kg of preferred body weight |
| V_{e spont} | < 60 mL/kg of preferred body weight per minute |
| Work Estimate | > 1.2 Joules/L |

The thresholds listed in Table 1 above are exemplary only and are not limiting.

The threshold monitor module **216** notifies the ventilator module **212** as soon as a key variable exceeds a threshold value or falls outside of a desired range. Further, in one embodiment, the threshold monitor module **216** times the SBT period. In this embodiment, the threshold monitor module **216** notifies the ventilator module **212** as soon as the SBT period ends. Additionally, the threshold monitor module **216** may store this information in storage **210** or send it for display on the display module **200.**

The ventilation module **212** may send commands to the ventilator **204.** In one embodiment, the ventilation module **212** utilizes ventilator information, patient information, inputted parameters and commands, and/or threshold monitoring module information to determine the proper ventilator commands. In one embodiment, the ventilator module **212** commands the medical ventilator **204** to initiate a SBT, return to previous ventilator settings, alter the predetermined amount of time for a SBT, end a SBT, change a breath type of a SBT, alter the parameters of a SBT, and/or alter ventilator settings. For example, if the predetermined amount of time for the SBT expires, the ventilation module **212** may command the medical ventilator **204** to return to the ventilator settings utilized before the initiation of the SBT. In another example, the ventilation module **212** may command the ventilator to change a SBT ventilator setting based on new user inputted information.

The user interface **214** of the SBT manger **202** allows a user to adjust SBT parameters, ventilator parameter, and patient information suitable for affecting a SBT during a SBT. In one embodiment, the support level, the oxygen percentage of the gas mixture, PEEP, trial period, and/or breath type of the ventilator can be adjusted by a user through the SBT manager **202.** In an alternative embodiment, the user interface **214** is a touch sensitive display. In the embodiment shown, the user interface **214** is a data entry station, such a keyboard. In one embodiment, the user interface **214** may generate smart prompts or ventilator setting recommendations or SBT protocols for a SBT based on patient and ventilator information, which are displayed by the display module **200.** In another embodiment, the user interface **214** may recommend the initiation of a SBT based on patient and ventilator information, which is displayed through the display module **200.** The user interface **214** sends all user commands and information to the ventilation module **212.** In one embodiment, displayed user interface information can provide for quick set-up and rapid activation of a SBT for an operator. Accordingly, the SBT manager **202** decreases the amount time necessary to program and/or initiate a SBT by a clinician compared to previously utilized SBT systems.

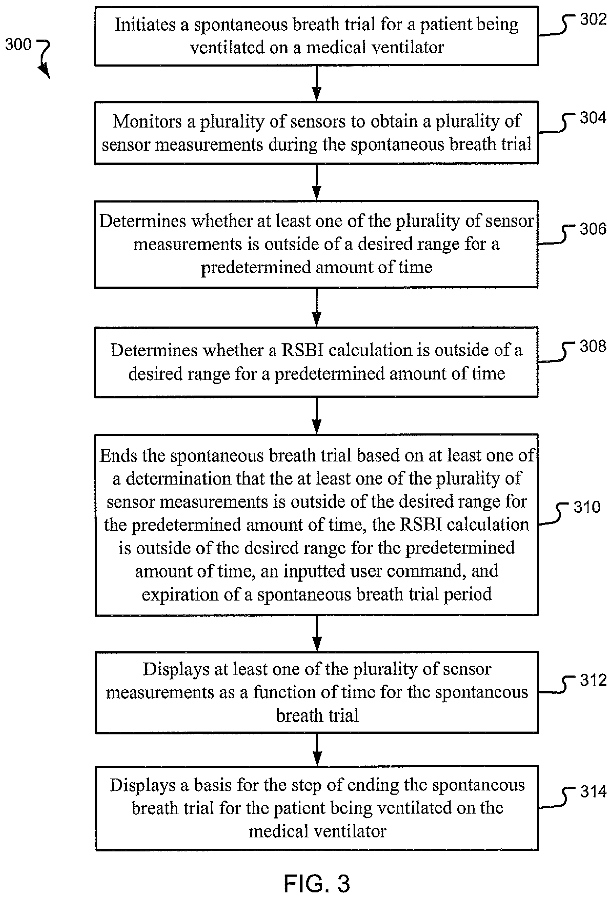

FIG. 3 represents an embodiment of a method for managing a spontaneous breathing trial in a medical ventilator **300.** In one embodiment, method **300** modifies the spontaneous breathing trial based on at least one of user inputted parameters and user inputted commands during operation of the spontaneous breathing trial. In another embodiment, method **300** recommends spontaneous breathing trial ventilator parameters to an operator for the patient based on at least of past and present ventilation information and past and present patient information. In this embodiment, the operator may choose to ignore recommended parameters, partially utilize recommended parameters, or fully utilize recommended parameters.

As illustrated, method **300** initiates a spontaneous breathing trial for a patient being ventilated on a medical ventilator **302.** In one embodiment, method **300** initiates the breathing trial based on user command. In another embodiment, method **300** initiates the breathing trial based on preconfigured conditions. In a further embodiment, method **300** initiates the breathing trial based on preset conditions entered or selected by the operator. In an additional embodiment, method **300** initiates the breathing trial based on an inputted user parameter. In one embodiment, the predetermined amount of time for the SBT is 30 minutes. In another embodiment, the predetermined amount of time for the SBT is 45 minutes. The previous embodiments are not meant to be limiting. Any suitable predetermined amount of time for a SBT may be utilized by method **300.**

Further, method **300** monitors a plurality of sensors to obtain a plurality of sensor measurements during the spontaneous breathing trial **304.** In one embodiment, method 300 monitors at least one of a breath frequency sensor, a spontaneous tidal volume (V_{t spont}) sensor, a spontaneous exhalation volume (V_{e spont}) sensor, a carbon dioxide elimination sensor, a SpO₂ sensor, and a heart rate sensor. In another embodiment, method 300 obtains at least one of a breath frequency, an RSBI, a spontaneous tidal volume (V_{t spont}), a spontaneous exhalation volume (V_{e spont}), a carbon dioxide elimination, a SpO₂, and a heart rate measurement. In another embodiment, the sensor measurements includes breath frequency and at least one of respiration rate, carbon dioxide elimination levels, spontaneous tidal volume, spontaneous exhalation volume, blood oxygen saturation level, and heart rate. In a further embodiment, the sensor measurements are breath frequency, spontaneous tidal volume, spontaneous exhalation volume, blood oxygen saturation level, and heart rate. The plurality of sensors may be located within the ventilator and/or may be external to the ventilator.

Next, method **300** determines whether at least one of the plurality of sensor measurements is outside of a desired range for a predetermined amount of time **306.** Further, method **300** determines whether a rapid shallow breathing index (RSBI) calculation is outside of a desired range for a predetermined amount of time **308.** The RSBI is calculated by utilizing an algorithm run by the processor.

The predetermined amount of time may be different for different measurements. Further, the predetermined amount of time may change when more than one measurement is outside of a desired range at one time. In one embodiment, the predetermined amount of time is 3 minutes. In another embodiment, the predetermined amount of time is 30 seconds. For example, in one embodiment, the RSBI calculation must exceed a desired range for 3 minutes unless another measurement is exceeded for time period of 30 seconds causing the desired RSBI violation time to shorten.

Method **300** ends the spontaneous breathing trial based on at least one of a determination that at least one of the plurality of sensor measurements is outside of the desired range for the predetermined amount of time, the RSBI calculation is outside of the desired range for the predetermined amount of time, an inputted user command, and expiration of a spontaneous breathing trial period **310.** In one embodiment, method **300** ends the spontaneous breathing trial based on at least one of the RSBI calculation, a breath frequency sensor measurement, a respiration rate measurement, a carbon dioxide elimination measurement, a spontaneous tidal volume measurement, a spontaneous exhalation volume measurement, a blood oxygen saturation measurement, and a heart rate measurement being outside the desired range for three minutes. In one embodiment, method **300** ends the spontaneous breathing trial based on the RSBI calculation and at least one of a respiration rate measurement, a carbon dioxide elimination measurement, a spontaneous tidal volume measurement, a breath frequency measurement, a spontaneous exhalation volume measurement, a blood oxygen saturation measurement, and a heart rate measurement being outside the desired range for three minutes. In another embodiment, method **300** ends the spontaneous breathing trial based on the RSBI calculation being outside the desired range for three minutes and at least one of a respiration rate measurement, a carbon dioxide elimination measurement, a breath frequency measurement, a spontaneous tidal volume measurement, a spontaneous exhalation volume measurement, a blood oxygen saturation level measurement, and a heart rate measurement being outside the desired range for about 5 seconds. In a further embodiment, method **300** ends the spontaneous breathing trial based on at least two of a respiration rate measurement, a carbon dioxide elimination measurement, a spontaneous tidal volume measurement, a spontaneous exhalation volume measurement, a blood oxygen saturation measurement, and a heart rate measurement being outside the desired range for one minute.

As shown, method **300** displays at least one of the plurality of sensor measurements as a function of time for the spontaneous breathing trial **312.** This display allows an operator to see trends in measurements for the SBT period. In one embodiment, method **300** displays at least one of spontaneous tidal volume, breath frequency, spontaneous exhalation volume, blood oxygen saturation level, carbon dioxide elimination levels, and heart rate as a function of time for the spontaneous breathing trial. In another embodiment, method 300 displays the RSBI calculation as a function of time for the spontaneous breathing trial. In this embodiment, method **300** displays the RSBI calculation as function time and at least one of spontaneous tidal volume, spontaneous exhalation volume, blood oxygen saturation level, breath frequency, carbon dioxide elimination levels, and heart rate as a function of time for the spontaneous breathing trial. In a further embodiment, method 300 displays the RSBI calculation, spontaneous tidal volume, spontaneous exhalation volume, blood oxygen saturation level, and heart rate as a function of time for the spontaneous breathing trial.

In yet another embodiment, method 300 displays at least two of spontaneous tidal volume, spontaneous exhalation volume, blood oxygen saturation level, carbon dioxide elimination levels, breath frequency, and heart rate as a function of time for the spontaneous breathing trial.

Further, method 300 displays a basis for the step of ending the spontaneous breathing trial for the patient being ventilated on the medical ventilator 314. In one embodiment, method 300 displays that the predetermined amount of time for the SBT expired as the basis for ending the spontaneous breathing trial. In another embodiment, method 300 displays that the basis for ending the spontaneous breathing trial was a user entered command. In a further embodiment, method 300 displays that the basis for ending the spontaneous breathing trial was that at least one of the plurality of sensor measurements was outside of the desired range for the predetermined amount of time and/or the RSBI calculation was outside of the desired range for the predetermined amount of time. In an additional embodiment, method 300 further displays at least one of breath type, pressure support level, oxygen percentage of the gas mixture, PEEP, for the spontaneous breathing trial, and the remaining amount of time for the spontaneous breathing trial period.

Numerous other changes may be made which will readily suggest themselves to those skilled in the art and which are defined in the appended claims. While various embodiments have been described for purposes of this disclosure, various changes and modifications may be made which are well within the scope of the present invention. Numerous other changes may be made which will readily suggest themselves to those skilled in the art and which are defined in the appended claims.

## Claims

1. A medical ventilator system, comprising:
a processor (56; 206);
a gas regulator (46) configured to be controlled by the processor (56; 206), the gas regulator (46) adapted to regulate a flow of gas from a gas supply (44) to a patient (24) via a patient circuit (30);
a breath frequency sensor configured to be controlled by the processor, the breath frequency sensor being adapted to measure breath frequency of the patient;
a spontaneous tidal volume sensor configured to be controlled by the processor, the spontaneous tidal volume sensor being adapted to measure spontaneous tidal volume of the patient;
a spontaneous exhalation volume sensor configured to be controlled by the processor, the spontaneous exhalation volume sensor being adapted to measure spontaneous exhalation volume of the patient;
a SpO₂ sensor configured to be controlled by the processor, the SpO₂ sensor being adapted to measure blood oxygen saturation level of the patient;
a heart rate sensor configured to be controlled by the processor, the heart rate sensor being adapted to measure heart rate of the patient;
a spontaneous breathing trial manager (60; 202) in communication with the breath frequency sensor, the spontaneous tidal volume sensor, the spontaneous exhalation volume sensor, the SpO₂ sensor, and the heart rate sensor, the spontaneous breathing trial manager (60; 202) being in communication with the processor (56; 206), the spontaneous breathing trial manager (60; 202) comprising
- monitoring key variables by receiving sensor measurements of the breath frequency sensor, the spontaneous tidal volume sensor, the spontaneous exhalation volume sensor, the SpO₂ sensor, and the heart rate sensor to obtain a plurality of sensor measurements during a spontaneous breathing trial, to determine whether at least one of the plurality of sensor measurements is outside of a desired range for a predetermined amount of time, and to determine whether a RSBI calculation is outside of a desired range for a predetermined amount of time, and
- a threshold monitor module (216) configured to determine if key variables are within a desired range or beyond a desired threshold for a predetermined amount of time; the key variables are any suitable ventilator or patient information that is an indicator of the patient's tolerance to the SBT; the key variables are monitored through the sensor measurements;
- a ventilation module (212) configured to end the spontaneous breathing trial based on a determination by the threshold monitor module (216) that at least one of the plurality of sensor measurements is outside of the desired range for a predetermined amount of time and the RSBI calculation is outside of the desired range for the predetermined amount of time;
a user interface (214) in communication with the processor (56; 206), the user interface (214) being in communication with the spontaneous breathing trial manager; and
a display module (200) configured to be controlled by the processor (56; 206), the display module adapted to display a RSBI and at least one of heart rate, blood oxygen saturation level, spontaneous tidal volume, and spontaneous exhalation volume of the patient as a function of time for the spontaneous breathing trial.

2. The medical ventilator system of claim 1, wherein the display module (59; 200) is adapted to further display at least one of breath type, pressure support level, oxygen percentage of a gas mixture, PEEP, for the spontaneous breathing trial, and the remaining amount of time for the spontaneous breathing trial,
and/or wherein the display module is adapted to further display the reason for ending the spontaneous breathing trial,
and/or wherein the spontaneous breathing trial manager (202) further includes the processor (206).

3. The medical ventilator system of claim 1, further comprising a respiration rate sensor configured to be controlled by the processor, the respiration rate sensor being adapted to measure respiration rate of the patient; and
wherein the display module (59; 200) is further adapted to display the respiration rate of the patient as a function of time for a spontaneous breathing trial.

4. The medical ventilator system of claim 1, further comprising a carbon dioxide elimination sensor configured to be controlled by the processor, the carbon dioxide elimination sensor being adapted to measure carbon dioxide elimination levels of the patient; and
wherein the display module (59; 200) is further adapted to display the carbon dioxide elimination levels of the patient as a function of time for the spontaneous breathing trial.

## Patentansprüche

1. Medizinisches Beatmungssystem, umfassend:
einen Prozessor (56; 206);
einen Gasregler (46), der konfiguriert ist, durch den Prozessor (56; 206) gesteuert zu werden, wobei der Gasregler (46) angepasst ist, um eine Gasströmung von einer Gaszufuhr (44) zu einem Patienten (24) über einen Patientenkreis (30) zu regeln;
einen Sensor für Atemfrequenz, der konfiguriert ist, durch den Prozessor gesteuert zu werden, wobei der Sensor für Atemfrequenz angepasst ist, um die Atemfrequenz des Patienten zu messen;
einen Sensor für spontanes Atemzugvolumen, der konfiguriert ist, durch den Prozessor gesteuert zu werden, wobei der Sensor für spontanes Atemzugvolumen angepasst ist, um das spontane Atemzugvolumen des Patienten zu messen;
einen Sensor für spontanes Ausatmungsvolumen, der konfiguriert ist, durch den Prozessor gesteuert zu werden, wobei der Sensor für spontanes Ausatmungsvolumen angepasst ist, um das spontane Ausatmungsvolumen des Patienten zu messen;
einen SpO₂-Sensor, der konfiguriert ist, durch den Prozessor gesteuert zu werden, wobei der SpO₂-Sensor angepasst ist, um das Blutsauerstoffsättigungsniveau des Patienten zu messen;
einen Herzschlagsensor, der konfiguriert ist, durch den Prozessor gesteuert zu werden, wobei der Herzschlagsensor angepasst ist, um den Herzschlag des Patienten zu messen;
einen Testmanager für spontane Atmung (60; 202), der in Verbindung mit dem Atemfrequenzsensor, dem Sensor für spontanes Atemzugvolumen, dem Sensor für spontanes Ausatmungsvolumen, dem SpO₂-Sensor und dem Herzschlagsensor steht, wobei der Testmanager für spontane Atmung (60; 202) in Verbindung mit dem Prozessor (56; 206) steht, wobei der Testmanager für spontane Atmung (60; 202) Folgendes umfasst:
- Überwachen der Schlüsselvariablen durch Empfangen von Sensormessungen des Atmungsfrequenzsensors, des Sensors für spontanes Atemzugvolumen, des Sensors für spontanes Ausatmungsvolumen, des SpO₂-Sensors und des Herzschlagsensors, um eine Vielzahl von Sensormessungen während eines spontanen Atmungstests zu erhalten, um zu bestimmen, ob zumindest eine der Vielzahl der Sensormessungen außerhalb eines gewünschten Bereichs für einen vorbestimmten Zeitraum liegt, und zu bestimmen, ob eine RSBI-Berechnung außerhalb eines gewünschten Bereichs für einen vorbestimmten Zeitraum liegt, und
- ein Schwellenwert-Überwachungsmodul (216), das konfiguriert ist, zu bestimmen, ob die Schlüsselvariablen innerhalb eines gewünschten Bereichs oder über einem gewünschten Schwellenwert für einen vorbestimmten Zeitraum liegen, wobei die Schlüsselvariablen beliebig geeignete Beatmungs- oder Patienteninformation sind, die ein Zeichen für die Toleranz des Patienten gegenüber dem SBT sind; wobei die Schlüsselvariablen durch die Sensormessungen überwacht werden;
- ein Beatmungsmodul (212), das konfiguriert ist, den spontanen Atmungstest zu beenden, basierend auf einer Bestimmung durch das Schwellenwert-Überwachsungsmodul (216), das zumindest eine der Vielzahl der Sensormessungen außerhalb des gewünschten Bereichs für einen vorbestimmten Zeitraum liegt und der RSBI-Berechnung außerhalb des gewünschten Bereichs für einen vorbestimmten Zeitraum liegt;
eine Benutzerschnittstelle (214), die in Verbindung mit dem Prozessor (56; 206) steht, wobei die Benutzerschnittstelle (214) in Verbindung mit dem Testmanager für spontane Atmung steht; und
ein Anzeigenmodul (200), das konfiguriert ist, um durch den Prozessor (56; 206) gesteuert zu werden, wobei das Anzeigenmodul angepasst ist, um einen RSBI und zumindest eines von Herzschlag, Blutsauerstoffsättigungsniveau, spontanes Atemzugvolumen und spontanes Ausatmungsvolumen des Patienten als eine Funktion der Zeit für den spontanen Atmungstest anzeigt.

2. Medizinisches Beatmungssystem nach Anspruch 1, wobei das Anzeigenmodul (59; 200) angepasst ist, um ferner zumindest eines von Atmungstyp, Druckunterstützungsniveau, Sauerstoffanteil eines Gasgemischs, PEEP, für den spontanen Atmungstest, und den restlichen Zeitraum für den spontanen Atmungstest anzuzeigen,
und/oder wobei das Anzeigenmodul angepasst ist, um ferner den Grund für die Beendigung des spontanen Atmungstests anzuzeigen,
und/oder wobei der Testmanager für spontane Atmung (202) ferner den Prozessor enthält (206).

3. Medizinisches Beatmungssystem nach Anspruch 1, ferner umfassend einen Atmungsfrequenzsensor, der konfiguriert ist, durch den Prozessor gesteuert zu werden, wobei der Atmungsfrequenzsensor angepasst ist, um die Atmungsfrequenz des Patienten zu messen; und
wobei das Anzeigenmodul (59; 200) ferner angepasst ist, um die Atmungsfrequenz des Patienten als eine Funktion der Zeit für den spontanen Atmungstest anzuzeigen.

4. Medizinisches Beatmungssystem nach Anspruch 1, ferner umfassend einen Sensor für Kohlenstoffdioxidelimination, der konfiguriert ist, durch den Prozessor gesteuert zu werden, wobei der Sensor für Kohlenstoffdioxidelimination angepasst ist, um das Kohlenstoffdioxideliminationsniveau des Patienten zu messen; und
wobei das Anzeigenmodul (59; 200) ferner angepasst ist, um die Kohlenstoffdioxideliminationsniveaus des Patienten als eine Funktion der Zeit für den spontanen Atmungstest anzuzeigen.

## Revendications

1. Système de ventilation médicale, comprenant :
un processeur (56 ; 206) ;
un régulateur de gaz (46) configuré pour être commandé grâce audit processeur (56 ; 206), le régulateur de gaz (46) étant conçu pour réguler une circulation de gaz à partir d'une alimentation de gaz (44) vers un patient (24) via un circuit de patient (30) ;
un capteur de fréquence respiratoire configuré pour être commandé par le processeur, le capteur de fréquence respiratoire étant conçu pour mesurer la fréquence respiratoire du patient ;
un capteur de volume courant spontané configuré pour être commandé par le processeur, le capteur de volume courant spontané étant conçu pour mesurer un volume courant spontané du patient ;
un capteur de volume expiratoire spontané configuré pour être commandé par le processeur, le capteur de volume expiratoire spontané étant conçu pour mesurer un volume expiratoire spontané du patient ;
un capteur de saturation en oxygène (SpO₂) configuré pour être commandé par le processeur, le capteur de saturation en oxygène (SpO₂) étant conçu pour mesurer le niveau de saturation en oxygène du sang du patient ;
un capteur de fréquence cardiaque configuré pour être commandé par le processeur, le capteur de fréquence cardiaque étant conçu pour mesurer la fréquence cardiaque du patient ;
un dispositif de gestion d'essai de ventilation spontanée (60 ; 202) en communication avec le capteur de fréquence respiratoire, le capteur de volume courant spontané, le capteur de volume expiratoire spontané, le capteur de saturation en oxygène (SpO₂), (SpO₂) et le capteur de fréquence cardiaque, le dispositif de gestion d'essai de ventilation spontanée (60 ; 202) étant en communication avec le processeur (56 ; 206), le dispositif de gestion d'essai de ventilation spontanée (60 ; 202) comprenant une étape consistant à
- surveiller des variables fondamentales grâce à une étape consistant à recevoir des mesures de capteur en provenance du capteur de fréquence respiratoire, du capteur de volume courant spontané, du capteur de volume expiratoire spontané, du capteur de saturation en oxygène (SpO₂), et du capteur de fréquence cardiaque afin d'obtenir une pluralité de mesures de capteur pendant un essai de ventilation spontanée, afin de déterminer si au moins une parmi la pluralité de mesures de capteur se situent à l'extérieur d'une plage souhaitée pendant une quantité prédéterminée de temps, et afin de déterminer si un calcul d'indice fréquence sur volume courant (RSBI) se situe à l'extérieur d'une plage souhaitée pendant une quantité prédéterminée de temps, et
- un module de surveillance de seuil (216) configuré pour déterminer si les variables fondamentales se situent à l'intérieur d'une plage souhaitée ou au-delà d'un seuil souhaité pendant une quantité prédéterminée de temps ; les variables fondamentales correspondant à toute information de patient ou de dispositif de ventilation appropriée qui constitue un indicateur de la tolérance du patient envers l'essai de ventilation spontanée (SBT) ; les variables fondamentales étant surveillées par l'intermédiaire des mesures de capteur ;
- un module de ventilation (212) configuré pour clore l'essai de ventilation spontanée en se basant sur une détermination, grâce au module de surveillance de seuil (216), qu'au moins une parmi la pluralité de mesures de capteur se situe à l'extérieur de la plage souhaitée pendant une quantité prédéterminée de temps et que le calcul RSBI se situe à l'extérieur de la plage souhaitée pendant la quantité prédéterminée de temps ;
une interface utilisateur (214) en communication avec le processeur (56 ; 206), l'interface utilisateur (214) étant en communication avec le dispositif de gestion d'essai de ventilation spontanée ; et
un module d'affichage (200) configuré pour être commandé grâce au processeur (56 ; 206), le module d'affichage étant conçu pour afficher un RSBI et au moins un parmi une fréquence cardiaque, un niveau de saturation en oxygène du sang, un volume courant spontané, et un volume expiratoire spontané du patient en fonction du temps pendant l'essai de ventilation spontanée.

2. Système de ventilation médicale selon la revendication 1, dans lequel le module d'affichage (59 ; 200) est conçu pour afficher en outre au moins un parmi un type de respiration, un niveau de soutien de pression, un pourcentage d'oxygène d'un mélange gazeux, une pression positive en fin d'expiration (PEEP), pendant l'essai de ventilation spontanée, et la quantité de temps restante pour l'essai de ventilation spontanée,
et/ou dans lequel le module d'affichage est conçu pour afficher en outre la raison de la clôture de l'essai de ventilation spontanée,
et/ou dans lequel le dispositif de gestion d'essai de ventilation spontanée (202) comprend en outre le processeur (206).

3. Système de ventilation médicale selon la revendication 1, comprenant en outre un capteur de fréquence respiratoire configuré pour être commandé par le processeur, le capteur de fréquence respiratoire étant conçu pour mesurer la fréquence respiratoire du patient ; et
dans lequel le module d'affichage (59 ; 200) est en outre conçu pour afficher la fréquence respiratoire du patient en fonction du temps pendant un essai de ventilation spontanée.

4. Système de ventilation médicale selon la revendication 1, comprenant en outre un capteur d'élimination de dioxyde de carbone configuré pour être commandé par le processeur, le capteur d'élimination de dioxyde de carbone étant conçu pour mesurer des niveaux d'élimination de dioxyde de carbone du patient ; et
dans lequel le module d'affichage (59 ; 200) est en outre conçu pour afficher les niveaux d'élimination de dioxyde de carbone du patient en fonction du temps pendant l'essai de ventilation spontanée.
